# EUROPEAN PATENT APPLICATION

(11) **EP 2 857 005 A1**
(43) Date of publication of application: **08.04.2015**
(21) Application number: 13187316.8
(22) Date of filing: 04.10.2013
(51) Int. Cl.: A61K 9/00, A61K 31/485

(54) **Method for manufacturing an ingestible film, apparatus for manufacturing an ingestible film, ingestible film and pharmaceutical dosage form comprising the same**

(71) Applicant: Hexal AG, 83607 Holzkirchen (DE)
(72) Inventor: Kohr, Thomas, 83607 Holzkirchen (DE)
(74) Representative: TBK

(57) **Abstract**

The present invention relates to a method for manufacturing an ingestible film, an apparatus for manufacturing an ingestible film, an ingestible film and a pharmaceutical dosage form comprising the same. The method comprises providing a matrix comprising at least a solvent and a polymer; a film forming step of forming a wet film from said matrix; a heating step of heating said wet film by radiation originating from a radiation source to obtain a heated wet film having a temperature of at least 22°C; and a drying step of drying said heated wet film using a drying means.

## Description

### Technical field

The present invention relates to a method for manufacturing an ingestible film, an ingestible film obtainable by the method, a pharmaceutical dosage form comprising the ingestible film, and an apparatus for drying a wet film usable in the method.

### Background of the invention

Ingestible films are known in the art as well suited for pharmaceutical dosage forms which include a pharmaceutically active compound. Such films are usually produced by providing a wet film from a film forming matrix comprising additives and a solvent and then drying the wet film.

In conventional drying techniques, such as disclosed in WO 2004/066986 A1, WO 2008/036299 A2 and US 8017150 B2, the wet film is dried using a high temperature air-bath by means of a drying oven, a drying tunnel, or a vacuum drier, or using radiation such as IR or microwave radiation.

However, the prior art techniques suffer from several drawbacks. In cases where radiation is used to dry the wet film, drying times are considerably long, leading to low production efficiency or a high amount of residual solvent. On the other hand, although air-drying can achieve short drying times, bubbles may be generated due to film formation in the initial stage of the drying, which leads to an undesirable appearance. Such film formation may lead to high amounts of residual solvent in the film as well.

These problems are aggravated when using film forming compositions having high viscosity. High viscosity of the film forming matrix, however, allows for production of very homogeneous (uniform) films.

### Disclosure of the invention

The present invention aims at providing a method for manufacturing an ingestible (edible) film overcoming the drawbacks of the prior art. Specifically, the present invention aims at providing an efficient method allowing the production of a homogeneous ingestible film having good appearance and low content of residual solvent. The present invention further relates to an ingestible film obtained by the method, a pharmaceutical dosage form comprising the ingestible film, and an apparatus for drying a wet film usable in the method.

The present invention is defined by a method according to claim 1, an ingestible film according to claim 9, a pharmaceutical dosage form according to claim 11, and an apparatus according to claim 12. Further beneficial developments are set forth in the dependent claims. Specifically, the present invention relates to one or more of the following items:
1. A method for manufacturing an ingestible film, the method comprising:
   providing a matrix comprising at least a solvent and a polymer;
   a film forming step of forming a wet film from said matrix;
   a heating step of heating said wet film by radiation originating from a radiation source to obtain a heated wet film having a temperature of at least 22°C; and
   a drying step of drying said heated wet film using a drying means.
2. The method according to item 1, wherein said radiation is IR radiation.
3. The method according to item 1 or 2, wherein said radiation source is provided above the wet film.
4. The method according to any one of items 1 to 3, wherein no solid material is provided between said radiation source and said wet film.
5. The method according to any one of items 1 to 4, wherein said radiation source is an apparatus directly converting electrical energy into radiation.
6. The method according to any one of items 1 to 5, wherein said radiation source is an IR dryer, an IR lamp or an IR panel.
7. The method according to any one of items 1 to 6, wherein the distance between said wet film and said radiation source is 1 to 100 cm, preferably 5 to 90 cm.
8. The method according to any one of items 1 to 7, wherein said wet film is heated by said radiation to a temperature of at least 25°C, preferably at least 40°C, preferably not more than 60°C, more preferably not more than 55°C, before said drying step.
9. The method according to any one of items 1 to 8, wherein said wet film is heated by said radiation to a temperature of from 22°C to 60°C before said drying step, preferably 22°C to 55°C.
10. The method according to any one of items 1 to 9, wherein said wet film is heated by said radiation to a temperature of from 25°C to 60°C before said drying step, preferably 25°C to 55°C.
11. The method according to any one of items 1 to 10, wherein said wet film is heated by said radiation such that a difference in temperature between the heated wet film and the temperature at the beginning of the drying step is 20°C or less, preferably 15°C or less, preferably 10°C or less, preferably 8°C or less, preferably 5°C or less, preferably 3°C or less, preferably 1°C or less.
12. The method according to any one of items 1 to 11, wherein said wet film is heated by said radiation for 0.1 to 10 minutes before said drying step.
13. The method according to any one of items 1 to 12, wherein said radiation source emits radiation at a power of 150 to 1500 W.
14. The method according to any one of items 1 to 13, wherein said radiation source provides a total power of 0.15 to 15 W/cm² towards said wet film before said drying step.
15. The method according to any one of items 1 to 14, wherein said radiation is further applied to said heated film during said drying step.
16. The method according to any one of items 1 to 15, wherein said drying means in said drying step is an oven.
17. The method according to any one of items 1 to 16, wherein said drying means in said drying step is hot air.
18. The method according to item 17, wherein said hot air has an air temperature of 40 to 120 °C.
19. The method according to item 17 or 18, wherein said hot air is applied from the top side, bottom side or top and bottom side of said film.
20. The method according to any one of items 1 to 19, wherein said film forming step is carried out at a film forming temperature below the boiling point of the solvent.
21. The method according to any one of items 1 to 20, wherein said film forming step is carried out at a film forming temperature of at least 18°C, preferably at least 20°C, preferably not more than 60°C, preferably not more than 40°C, preferably not more than 25°C.
22. The method according to any one of items 1 to 21, wherein said film forming step is carried out at room temperature (21°C ± 5°C).
23. The method according any one of items 1 to 22, wherein said wet film is dried at a drying temperature of 100°C or less, preferably 90°C or less, further preferably 80°C or less, in said drying step.
24. The method according to item 23, wherein said drying temperature is 30°C or more, preferably 40°C or more, preferably 50°C or more, preferably 70°C or more.
25. The method according to any one of items 1 to 24, wherein said ingestible film is a self-supporting film.
26. The method according to any one of items 1 to 25, wherein said matrix has a dynamic viscosity of at least 1500 mPa*s, as measured with a plate disc system and an agitation of 100 1/s.
27. The method according to item 26, wherein said dynamic viscosity is at least 2000 mPa*s, preferably at least 2500 mPa*s.
28. The method according to item 26 or 27, wherein said dynamic viscosity is not more than 100000 mPa*s, preferably not more than 10000 mPa*s.
29. The method according to any one of items 1 to 28, wherein said solvent comprises at least 10% v/v, at least 15% v/v, or at least 20% v/v, preferably 22% to 28% v/v of water.
30. The method according to any one of items 1 to 29, wherein said solvent comprises at least 50% v/v, at least 70% v/v, or 90 % v/v or less, preferably 72% to 78% v/v of an organic solvent.
31. The method according to any one of items 1 to 30, wherein said solvent comprises acetone.
32. The method according to any one of items 1 to 31, wherein said matrix further comprises a pharmaceutically active ingredient.
33. The method according to item 32, wherein said pharmaceutically active ingredient is buprenorphine hydrochloride and/or naloxone hydrochloride dihydrate.
34. The method according to any one of items 1 to 33, wherein said matrix further comprises at least one additive.
35. The method according to item 34, wherein said additive is at least a flavoring substance, preferably lemon oil.
36. The method according to any one of items 1 to 35, wherein said wet film has a thickness of 100 to 1000 µm.
37. The method according to any one of items 1 to 36, wherein said ingestible film has a thickness of 50 to 1000 µm.
38. The method according to any one of items 1 to 37, further comprising a step of dividing the dried ingestible film into individual sub-units.
39. An ingestible film formed from a matrix comprising at least a solvent and a polymer, the ingestible film being obtainable by a method comprising the steps of
   forming a wet film from said matrix,
   heating said wet film by radiation originating from a radiation source to obtain a heated wet film having a temperature of at least 22°C; and
   drying said heated wet film using drying means.
40. The indigestible film according to item 39, further comprising an active ingredient.
41. A pharmaceutical dosage form comprising the ingestible film according to item 39 or 40.
42. An apparatus for drying a wet film, the apparatus comprising at least
   a transporting support for transporting said wet film;
   a radiation zone comprising a radiation source for heating said wet film; and
   a drying zone comprising a drying means for drying said heated wet film.
43. The apparatus according to item 42, wherein said drying means is provided in addition to said radiation source.
44. The apparatus according to item 42 or 43, wherein said radiation zone and said drying zone are separated from each other.
45. The apparatus according to item 44, wherein said drying zone is located behind said radiation zone in the direction of film movement when operating the apparatus.
46. The apparatus according to item 42 or 43, wherein said radiation zone is integrated in said drying zone.
47. The apparatus according to any one of items 42 to 46, wherein said apparatus is adapted to transport said wet film through said radiation zone and subsequently through said drying zone.
48. The apparatus according to any one of items 42 to 47, wherein said drying means is an oven.
49. The apparatus according to any one of items 42 to 48, which is adapted to carry out a method comprising:
   providing a matrix comprising at least a solvent and a polymer;
   a film forming step of forming a wet film from said matrix;
   a heating step of heating said wet film by radiation originating from said radiation source to obtain a heated wet film having a temperature of at least 22°C; and
   a drying step of drying said heated wet film using said drying means.
50. The apparatus according to any one of items 42 to 49, wherein said radiation source is an IR radiation source.
51. The apparatus according to any one of items 42 to 50, wherein said radiation source is provided above said transporting support.
52. The apparatus according to any one of items 42 to 51, wherein said apparatus is constructed such that no solid material is provided between said radiation source and said wet film supported by said transporting support.
53. The apparatus according to any one of items 42 to 52, wherein said radiation source is an apparatus directly converting electrical energy into radiation.
54. The apparatus according to any one of items 42 to 53, wherein said radiation source is an IR dryer, an IR lamp or an IR panel.
55. The apparatus according to any one of items 42 to 54, wherein the distance between said transporting support and said radiation source is 1 to 100 cm, preferably 5 to 90 cm.
56. The apparatus according to any one of items 42 to 55, wherein said apparatus is adapted to heat said wet film by said radiation to a temperature of at least 25°C, preferably at least 40°C, preferably not more than 60°C, more preferably not more than 55°C, before said drying step.
57. The apparatus according to any one of items 42 to 56, wherein said apparatus is adapted to heat said wet film by said radiation to a temperature of from 22°C to 60°C before said drying step, preferably 22°C to 55°C. Preferably said apparatus is adapted to heat said wet film by said radiation to a temperature of from 25°C to 60°C before said drying step, most preferably 25°C to 55°C.
58. The apparatus according to any one of items 42 to 57, wherein said apparatus is adapted to heat said wet film by radiation such that a difference in the temperature between the heated wet film and the temperature at the beginning of the drying step is 20°C or less, preferably 15°C or less, preferably 10°C or less, preferably 8°C or less, preferably 5°C or less, preferably 3°C or less, preferably 1°C or less.
59. The apparatus according to any one of items 42 to 58, wherein said apparatus is adapted to heat said wet film by said radiation for 0.1 to 10 minutes before said drying step.
60. The apparatus according to any one of items 42 to 59, wherein said radiation source is adapted to emit radiation at a power of 150 to 1500 W.
61. The apparatus according to any one of items 42 to 60, wherein said radiation source is adapted to provide a total power of 0.15 to 15 W/cm² towards said wet film before said drying step.
62. The apparatus according to any one of items 42 to 61, wherein said apparatus is adapted to further apply said radiation to said heated film during said drying step.
63. The apparatus according to any one of items 42 to 62, wherein said drying means is hot air.
64. The apparatus according to item 63, wherein said apparatus is adapted such that said hot air has an air temperature of 40 to 120 °C.
65. The apparatus according to item 63 or 64, wherein said apparatus is adapted such that said hot air is applied from the top side, bottom side or top and bottom side of said film.
66. The apparatus according to any one of items 42 to 65, wherein said apparatus is adapted such that the wet film is dried at a drying temperature of 100°C or less, preferably 90°C or less, further preferably 80°C or less in said drying zone.
67. The apparatus according to item 66, wherein said drying temperature is 30°C or more, preferably 40°C or more, preferably 50°C or more, more preferably 70°C or more.
68. The apparatus according to any one of items 42 to 67, further comprising a film forming zone for performing a film forming step of forming a matrix comprising at least a solvent and a polymer into a wet film.
69. The apparatus according to item 68, wherein said apparatus is adapted such that said film forming step is carried out at a film forming temperature below the boiling point of the solvent.
70. The apparatus according to item 68 or 69, wherein said apparatus is adapted such that said film forming step is carried out at a film forming temperature of at least 18°C, preferably at least 20°C, preferably not more than 60°C, preferably not more than 40°C, preferably not more than 25°C.
71. The apparatus according to any one of items 68 to 70, wherein said apparatus is adapted such that said film forming step is carried out at room temperature (21°C ± 5°C).
72. The apparatus according to any one of items 68 to 71, wherein said matrix has a dynamic viscosity of at least 1500 mPa*s, as measured with a plate disc system and an agitation of 100 1/s.
73. The apparatus according to item 72, wherein said dynamic viscosity is at least 2000 mPa*s, preferably at least 2500 mPa*s.
74. The apparatus according to items 72 or 73, wherein said dynamic viscosity is not more than 100000 mPa*s, preferably not more than 10000 mPa*s.
75. The apparatus according to any one of items 68 to 74, wherein said solvent comprises at least 10% v/v, at least 15% v/v, or at least 20% v/v, preferably 22% to 28% v/v of water.
76. The apparatus according to any one of items 68 to 75, wherein said solvent comprises at least 50% v/v, at least 70% v/v, or at least 90 % v/v or less, preferably 72% to 78% v/v of an organic solvent.
77. The apparatus according to any one of items 68 to 76, wherein said solvent comprises acetone.
78. The apparatus according to any one of items 68 to 77, wherein said matrix further comprises a pharmaceutically active ingredient.
79. The apparatus according to item 78, wherein said pharmaceutically active ingredient is buprenorphine hydrochloride and/or naloxone hydrochloride dihydrate.
80. The apparatus according to any one of items 68 to 79, wherein said matrix further comprises at least one additive.
81. The apparatus according to item 80, wherein said additive is at least a flavoring substance, preferably lemon oil.
82. The apparatus according to any one of items 68 to 81, wherein said apparatus further has a matrix formation zone for forming said matrix comprising at least said solvent and said polymer.
83. The apparatus according to any one of items 42 to 82, wherein said apparatus is adapted to produce a self-supporting film.
84. The apparatus according to any one of items 42 to 83, wherein said apparatus is adapted to dry a wet film having a thickness of 100 to 1000 µm.
85. The apparatus according to any one of items 42 to 84, wherein said apparatus is adapted to produce an ingestible film having a thickness of 50 to 1000 µm.
86. The apparatus according to any one of items 43 to 85, further comprising a dividing zone for dividing the dried ingestible film into individual sub-units.

### Detailed description of the invention

The special features and advantages of the method and the apparatus of the present invention and their impact on the ingestible film and the pharmaceutical dosage form of the present invention will now be discussed in detail. Unless stated otherwise, all preferred embodiments of the present invention, and in particular all numerical limitations, can be combined with each other. For example, it is particularly preferred that the method of the present invention employs an IR radiation source as a radiation source in combination with hot air drying as a drying means so as to produce an ingestible film containing a pharmaceutically active ingredient and optional pharmaceutically acceptable additives.

Unlike the conventional approaches, the present invention uses a combination of pre-heating a wet film by radiation followed by a subsequent drying step to produce an ingestible (edible) film. Since the wet film is heated before initiation of the drying step, formation of bubbles can be avoided. This is assumed to be due to the fact that heating by radiation allows uniform heating of the complete wet film, i.e. without formation of a temperature gradient within the wet film and without skin formation on the wet film at an initial stage of the drying step. Furthermore, since heating is performed after the film forming step, a low temperature can be maintained during the film forming step so that the film forming step is not affected by undesired heating. This allows the production of a film having high homogeneity.

In the present invention, the drying means is preferably provided in addition to the radiation source. This means that the drying means may be a separate apparatus. The drying means may further be different in the type of delivering energy to the heated wet film, as compared to the radiation source.

The drying means may be a radiation source for drying such as an IR dryer as well. In this case, however, the drying means differs from the radiation source in the heating step at least in its power output and/or distance from the wet film.

It is further possible that the drying means is no radiation source, meaning that it is not the main function of the heating source to provide radiation. An oven can be given as an example of such a drying means. This drying means may dry the heated wet film by bringing the heated wet film in direct contact with a hot medium or material such as hot air or a hot transporting support.

The method and apparatus of the present invention can be provided in two main constitutions.

As a first constitution, the drying zone is provided after the heating zone. In this constitution, no drying within the above meaning is performed during the heating step, i.e. no drying by contact with a hot medium is performed in addition to the exposure to radiation during the heating step. In particular, no flow of hot air is provided during the heating step. This allows for defined heating and drying conditions.

As a second constitution, the heating zone is integrated in the drying zone. This integration means that the radiation is provided either during the complete drying step or at least at the beginning of the drying step but not during the complete drying step. In this constitution, a rapid heating can be achieved in the heating step while the present invention still provides significant improvement over methods employing hot air drying only or IR drying under constant conditions.

In the above-mentioned second constitution, it is preferred that at least one drying means other than a radiation drying means is provided, optionally in addition to a radiation drying means.

The radiation preferably is IR radiation. Using IR radiation, a highly uniform heating of even thick films can be achieved with low costs.

Although the radiation source may be placed above, below and besides the wet film, the radiation source is preferably provided above and besides the wet film and particularly preferably above the wet film. Herein, "above/below" means "above/below in the direction of gravity", respectively. "Besides" means that the radiation source is neither above nor below the wet film. "Above" and "below" is preferably directly above and directly below, e.g. such that the radiation emitting surface/area of the radiation source is parallel to the film surface.

From the viewpoint of handling, it is desirable to provide the radiation source above the wet film. If the radiation source is below the wet film, it may be necessary to provide a support, i.e. a solid material, which is transparent for the radiation between the wet film and the radiation source. Such a material is difficult to handle, especially when considering that the wet film usually needs to be transported over the support or by the support. In the latter case, a flexible support would be needed. Further, such a material will usually not be 100% transparent for the radiation, leading to a loss in efficiency. Alternatively, the wet film can be exposed to the radiation from the bottom without a support. However, this may lead to problems with the stability of the film and is thus not desired from the viewpoint of handling either.

The radiation source is particularly preferably an apparatus directly converting electrical energy into radiation in view of the degree of efficiency of converting the electrical energy into radiation. An IR dryer, an IR lamp or an IR panel are preferably used. Among those, an IR dryer is most preferable, because it can provide uniform IR radiation.

The distance between said wet film and said radiation source is preferably 1 to 100 cm, more preferably 5 to 90 cm, in order to ensure high transfer efficiency. Since the wet film has only a very small thickness, the distance between the wet film and the radiation source is roughly the same as the distance between the transporting support and the radiation source. The term "distance" herein means the shortest distance between two objects.

The wet film is preferably heated by the radiation to a temperature of at least 22°C, preferably at least 25°C, before said drying step. The heating temperature is preferably not more than 60°C, more preferably not more than 45°C, before said drying step. That is, it is preferred that the temperature of the film is within the above specified ranges before initiation of the drying step. In particular it is preferred that the difference in temperature between the heated wet film and the temperature at the beginning of the drying step is 20°C or less, preferably 15°C or less, preferably 10°C or less, preferably 8°C or less, preferably 5°C or less, preferably 3°C or less, preferably 1°C or less. If the wet film is heated up to the above temperatures before the drying step, a high drying efficiency with low degrees of residual solvent can be achieved while the resulting film is free of bubbles. Further, since the heating is performed after the formation of the wet film, high viscosity can be maintained during formation of the wet film, allowing for high homogeneity of the resulting edible film.

The wet film is preferably heated by said radiation for at least 0.1 minutes before said drying step. Heating times of less than 10 minutes, preferably less than 5 minutes are desirable in view of production efficiency.

The radiation source preferably emits radiation at a power of 150 to 1500 W in order to ensure sufficient heating in short time while avoiding an excessive raise in temperature or formation of hot spots. For this reason, it is particularly preferable that the radiation source provides a total power of 0.15 to 15 W/cm² towards said wet film receives before said drying step.

The radiation may further be applied to the heated film during said drying step. Using two different heating/drying sources at the same time can increase the drying efficiency and lead to more uniform heating due to the additional use of a radiation source.

The drying means in the drying step may be an oven. Specifically, the wet film is preferably dried mainly by means of hot air within an oven which is in contact with the heated wet film. The drying may further be assisted by a flow of hot air. The air flow may be provided from above the heated wet film or from below the heated wet film, optionally via a support. Preferably, the hot air is applied from the top and bottom side of said film so as to avoid formation of a dry film on top of the heated wet film (skin formation), and thus avoid formation of bubbles. The hot air may have an air temperature of 40 to 120 °C in order to assure acceptable processing times while avoiding excessive heating of the heated wet film and thus avoiding degradation of an active ingredient, for example.

The film forming step should carried out at a film forming temperature below the boiling point of the solvent. Preferably, the film forming temperature is about room temperature, i.e. 21°C±5°C.

A drying temperature in the drying step 100°C or less, preferably 90°C or less, further preferably 80°C or less can be employed. The drying temperature is preferably 30°C or more, preferably 50°C or more, more preferably 70°C or more. The drying temperature herein is the temperature of the drying means (inside the oven, temperature of the hot air, etc.).

In the present invention, the film temperature at the beginning of the drying step is the film temperature when the drying starts. When the heating zone is separated from the drying zone e.g. by a wall having a slit, it corresponds to the temperature at the position of the slit. When the heating zone is integrated in the drying zone, the drying step begins at the end of the heating zone, i.e. where the radiation power changes (or drops to 0). The temperature at the end of the drying step is the film temperature directly before leaving the drying zone.

The ingestible film may be a self-supporting film due to ease of handling and production.

The matrix used for forming the wet film preferably has a dynamic viscosity of at least 1500 mPa*s, as measured with a plate disc system and an agitation of 100 1/s. Herein, the dynamic viscosity is the dynamic viscosity at room temperature. The dynamic viscosity is suitably at least 2000 mPa*s, preferably at least 2500 mPa*s. Using a matrix having high viscosity facilitates formation of a homogeneous film. Further, since the wet film is pre-heated before drying, high viscosity matrixes can be used while avoiding formation of bubbles. The dynamic viscosity is preferably not more than 100000 mPa*s, more preferably not more than 10000 mPa*s.

The solvent suitably comprises at least 10% v/v, at least 15% v/v, or at least 20% v/v, preferably 22% - 28% v/v of water. A water content of about 25% v/v is particularly preferable. Further, the solvent suitably comprises at least 50% v/v, at least 70% v/v, or at least 90 % v/v or less, preferably 72% - 78% v/v of an organic solvent. A content of organic solvent of about 75% v/v is particularly preferable. The solvent may comprise acetone as an organic solvent.

Most preferably, the matrix further comprises an active ingredient. Accordingly, the ingestible film comprises the active ingredient as well. As a matter of course, the processing conditions should be adjusted such that the active ingredient is not or not significantly degraded by the heating and drying procedure.

Examples of the active ingredient include, without limitation pharmaceutical and cosmetic actives, drugs, medicaments, proteins, antigens or allergens such as ragweed pollen, spores, microorganisms, seeds, mouthwash components, flavors, fragrances, enzymes, preservatives, sweetening agents, colorants, spices, vitamins and combinations thereof.

A wide variety of medicaments, bioactive active substances and pharmaceutical compositions may be included in the film of the present invention. Examples of useful drugs include ace-inhibitors, antianginal drugs, anti-arrhythmias, antiasthmatics, anti-cholesterolemics, analgesics, anesthetics, anti-convulsants, anti-depressants, anti-diabetic agents, anti-diarrhea preparations, antidotes, anti-histamines, anti-hypertensive drugs, anti-inflammatory agents, anti-lipid agents, anti-manics, anti-nauseants, anti-stroke agents, anti-thyroid preparations, anti-tumor drugs, anti-viral agents, acne drugs, alkaloids, amino acid preparations, anti-tussives, anti-uricemic drugs, anti-viral drugs, anabolic preparations, systemic and non-systemic anti-infective agents, anti-neoplasties, antiparkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, antihypertensive drugs, vasoconstrictors, migraine treatments, antibiotics, tranquilizers, antipsychotics, anti-tumor drugs, anti-coagulants, anti-thrombotic drugs, hypnotics, anti-emetics,anti-nauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

Examples of medicating active ingredients contemplated for use in the present invention include antacids, H₂-antagonists, and analgesics. For example, antacid dosages can be prepared using the ingredients calcium carbonate alone or in combination with magnesium hydroxide, and/or aluminum hydroxide. Moreover, antacids can be used in combination with H₂-antagonists.

Analgesics include opiates and opiate derivatives, such as buprenorphine, oxycodone (available as Oxycontin^{®}), oxymorphone, hydrocodone, hydromorphone, ibuprofen, aspirin, acetaminophen, and combinations thereof. Opiates may optionally include antagonists, preferably naloxone. In one embodiment opiats may optionally include caffeine.

Other preferred drugs for other preferred active ingredients for use in the present invention include anti-diarrheals such as immodium AD, anti-histamines, antitussives, decongestants, vitamins, and breath fresheners. Common drugs used alone or in combination for colds, pain, fever, cough, congestion, runny nose and allergies, such as acetaminophen, chlorpheniramine maleate, dextromethorphan, pseudoephedrine HCl and diphenhydramine may be included in the film compositions of the present invention.

Also contemplated for use herein are anxiolytics such as alprazolam (available as Xanax^{®}); anti-psychotics such as clozopin (available as Clozaril^{®}), aripiprazole, olanzapine, donepezil and haloperidol (available as Haldol^{®}); non-steroidal antiinflammatories (NSAID's) such as dicyclofenacs (available as Voltaren^{®}) and etodolac (available as Lodine^{®}), anti-histamines such as loratadine (available as Claritin^{®}), astemizole (available as Hismanal(TM)), nabumetone (available as Relafen^{®}), and Clemastine (available as Tavist^{®}); anti-emetics such as granisetron hydrochloride (available as Kytril^{®}) and nabilone (available as Cesamet(TM)); bronchodilators such as Bentolin^{®}, albuterol sulfate (available as Proventil^{®}); antidepressants such as fluoxetine hydrochloride (available as Prozac^{®}), sertraline hydrochloride (available as Zoloft^{®}), and paroxtine hydrochloride (available as Pail^{®}); anti-migraines such as Imigra^{®}, ACE-inhibitors such as enalaprilat (available as Vasotec^{®}), captopril (available as Capoten^{®}) and lisinopril (available as Zestril^{®}); anti- Alzheimer's agents, such as nicergoline; and Ca<H>-antagonists such as nifedipine (available as Procardia^{®} and Adalat^{®}), and verapamil hydrochloride (available as Clan^{®}).

Erectile dysfunction therapies include, but are not limited to, drugs for facilitating blood flow to the penis, and for effecting autonomic nervous activities, such as increasing parasympathetic (cholinergic) and decreasing sympathetic (adrenersic) activities. Useful non-limiting drugs include sildenafil, such as Viagra^{®}, tadalafil, such as Cialis^{®}, vardenafil, apomorphine, such as Uprima^{®}, yohimbine hydrochloride such as Aphrodyne^{®}, and alprostadil such as Caverject^{®}.

H₂-antagonists which are contemplated for use in the present invention include cimetidine, ranitidine hydrochloride, famotidine, nizatidien, ebrotidine, mifentidine, roxatidine, pisatidine and aceroxatidine.

Active antacid ingredients include, but are not limited to, the following: aluminum hydroxide, dihydroxyaluminum aminoacetate, aminoacetic acid, aluminum phosphate, dihydroxyaluminum sodium carbonate, bicarbonate, bismuth aluminate, bismuth carbonate, bismuth subcarbonate, bismuth subgallate, bismuth subnitrate, bismuth subsilysilate, calcium carbonate, calcium phosphate, citrate ion (acid or salt), amino acetic acid, hydrate magnesium aluminate sulfate, magaldrate, magnesium aluminosilicate, magnesium carbonate, magnesium glycinate, magnesium hydroxide, magnesium oxide, magnesium trisilicate, milk solids, aluminum mono-ordibasic calcium phosphate, tricalcium phosphate, potassium bicarbonate, sodium tartrate, sodium bicarbonate, magnesium aluminosilicates, tartaric acids and salts.

The pharmaceutically active agents employed in the present invention may include allergens or antigens, such as , but not limited to, plant pollens from grasses, trees, or ragweed; animal danders, which are tiny scales shed from the skin and hair of cats and other furred animals; insects, such as house dust mites, bees, and wasps; and drugs, such as penicillin.

An anti-oxidant may also be added to the film to prevent the degradation of an active, especially where the active is photosensitive.

Cosmetic active agents may include breath freshening compounds like menthol, other flavors or fragrances, especially those used for oral hygiene, as well as actives used in dental and oral cleansing such as quaternary ammonium bases. The effect of flavors may be enhanced using flavor enhancers like tartaric acid, citric acid, vanillin, or the like.

The matrix may comprise at least one (pharmaceutically acceptable) additive, as needed. The additive may be at least a flavoring substance. Lemon oil is particularly preferred as a flavoring substance.

Also color additives can be used in preparing the films. Such color additives include food, drug and cosmetic colors (FD&C), drug and cosmetic colors (D&C), or external drug and cosmetic colors (Ext. D&C). These colors are dyes, their corresponding lakes, and certain natural and derived colorants. Lakes are dyes absorbed on aluminum hydroxide.

Preferably, the wet film has a thickness of 100 to 1000 µm. The (dry) ingestible film preferably has a thickness of 50 to 1000 µm. This thickness allows easy handling during production, packaging and administration to a patient.

The method of the present invention preferably comprises a step of dividing the dried film into individual sub-units. Thus, the apparatus preferably provides a zone for dividing the dried film. The film is preferably divided into sub-units corresponding to an individual dosage forms ready for administration to a patient/person. The film may, however, be divided into larger sub-units having perforations allowing the patient/person to accurately divide the larger sub-units into sub-units corresponding to an individual dosage form.

The ingestible film of the present invention is obtainable by the method according to present invention. Therefore, the ingestible film has a low content of residual solvent and a good appearance. Good appearance is particularly important for pharmaceutical dosage forms which are the main application of the ingestible film of the present invention. That is, film having good appearance, specifically a film free of bubbles, leads to higher acceptance by the patient, and thus to higher compliance. In order to be well suited as a pharmaceutical dosage form, the ingestible film is preferably water soluble.

The apparatus of the present invention is preferably adapted to carry out the method of the present invention. Specifically, the apparatus is adapted to carry out at least the heating step and the drying step of the method of the present invention.

Thus, the apparatus comprises at least a transporting support for transporting the wet film; a radiation zone comprising a radiation source for heating the wet film; and a drying zone comprising a drying means for drying the heated wet film to obtain a dry film, wherein said drying means is different from said radiation source and said apparatus is adapted to transport said wet film through said radiation zone and subsequently through said drying zone.

It is preferable that the radiation zone and the drying zone are separated from each other. This separation can avoid undesired effects of the drying means in the radiation zone. Specifically, the apparatus is preferably adapted such that substantially no heat or hot material from said drying means reaches said radiation zone. Separation can be ensured by a solid build construction which preferably contains heat insulation. For example, transporting of the wet film can be carried out through a slit of suitable size in the solid construction. Further insulation can be achieved by directing air flow in the drying means to form an insulation layer of (hot) air, e.g. an air curtain. Nevertheless, heating by radiation can be continued during the drying step.

The apparatus may further comprise a wet film preparation zone for preparing a wet thin film from a matrix comprising a polymer and a solvent. The apparatus may just as well comprise a matrix formation zone for forming the matrix, preferably comprising a mixing means.

The apparatus may preferably be adapted to carry out any of the method steps described herein. As such the apparatus may comprise a processing unit which is programmed to control the apparatus such that the method is carried out automatically or semi-automatically. In order to allow full-automatic control, the apparatus will usually comprise one or more sensors, where necessary.

Although the invention has been described in view of its preferred embodiments above, the invention is not limited thereto. That is, the invention can be carried out using additional ingredients for producing the ingestible film by the method and the apparatus of the present invention.

The matrix for preparing the wet film comprises at least a solvent and a polymer.

Preferably, the matrix and the resulting ingestible film include a water soluble polymer composition. The matrix may include at least one water soluble polymer and may include other hydrophilic materials. The matrix may also include water swellable or water insoluble polymers, if desired. Further, the matrix may include a hydrophilic material selected from a saccharide-based polymer, a non-saccharide-based polymer, a sugar alcohol and combinations thereof.

The saccharide-based polymer may be cellulose or a cellulose derivative. Specific examples of useful saccharide-based, water soluble polymers include polydextrose, pullulan, hydroxypropylmethyl cellulose (HPMC, hypromellose), hydroxyethyl cellulose (HPC), hydroxypropyl cellulose, carboxymethyl cellulose, sodium aginate, xanthan gum, tragancanth gum, guar gum, acacia gum, arabic gum, starch, gelatin, and combinations thereof. Hypromellose and polydextrose are preferably used for self-supporting ingestible films.

Examples of non-saccharide based, water soluble polymers include polyethylene oxide, polyvinylpyrrolidone, polyvinyl alcohol, polyethylene glycol, polyacrylic acid, methylmethacrylate copolymer, carboxyvinyl copolymers, and combinations thereof, wherein a matrix containing polyethylene oxide or a mixture of polyethylene oxides having different molecular weights is preferably used.

The sugar alcohol may be selected from erythritol, sorbitol and xylitol.

Specific examples of useful water insoluble polymers include ethyl cellulose, hydroxypropyl ethyl cellulose, cellulose acetate phthalate, hydroxypropyl methyl cellulose phthalate and combinations thereof. The matrix may further include polyethylene oxide, alone or in combination with other hydrophilic materials.

Preferably, the matrix includes polyethylene oxide in combination with a cellulosic polymer. For example, the matrix may include a combination of polyethylene oxide and carboxymethyl cellulose, a combination of polyethylene oxide and hydroxypropyl cellulose, or a combination of polyethylene oxide and hydroxypropylmethyl cellulose. A combination of polyethylene oxide, hydroxypropylmethyl cellulose and polydextrose may be used as well.

Other polymers useful for incorporation into the matrix of the present invention include biodegradable polymers, copolymers, block polymers and combinations thereof. Among the known useful polymers or polymer classes are: polyglycolic acid (PGA), polylactic acid (PLA), polydioxanoes, polyoxalates, poly(α-esters), polyanhydrides, polyacetates, polycaprolactones, poly(orthoesters), polyamino acids, polyaminocarbonates, polyurethanes, polycarbonates, polyamides, poly(alkyl cyanoacrylates), and mixtures and copolymers thereof. Additional useful polymers include, stereopolymers of L- and D-lactic acid, copolymers of bis(p-carboxyphenoxy) propane acid and sebacic acid, sebacic acid copolymers, copolymers of caprolactone, polylactic acid/polyglycolic acid/polyethyleneglycol copolymers, copolymers of polyurethane and polylactic acid, copolymers of polyurethane and polylactic acid, copolymers of α-amino acids, copolymers of α-amino acids and caproic acid, copolymers of α-benzyl glutamate and polyethylene glycol, copolymers of succinate and polyglycols, polyphosphazene, polyhydroxy-alkanoates and mixtures thereof.

Although a variety of different polymers may be used, it is desired to select polymers to provide a desired viscosity of the matrix prior to heating. For example, if the active agent or other components are not soluble in the selected solvent, a polymer that will provide a greater viscosity is desired to assist in maintaining uniformity. On the other hand, if all components are soluble in the solvent, a polymer that provides a lower viscosity may be preferred in some cases.

The matrix may further comprise optional components such as active ingredients as recited above, a flavoring agent, an anti-foaming agent, an excipient, a lubricant, a buffering agent, a stabilizer, a blowing agent, a pigment, a coloring agent, a filler, a bulking agent, a sweetening agent, a fragrance, a release modifier, a adjuvant, a plasticizer, a flow accelerator, a mold release agent, a polyol, a granulating agent, a diluent, a binder, a buffer, an absorbent, a glidant, an adhesive, an anti-adherent, an acidulant, a softener, a demulcent, a surfactant, an emulsifier, an elastomer and mixtures thereof.

In order to prevent the formation of air bubbles in the ingestible film of the present invention, the matrix formation step or a subsequent step of mixing the formed matrix can be performed under vacuum. However, as soon as the mixing is completed, and the matrix is returned to the normal atmosphere condition, air will be re-introduced into or contacted with the matrix. In many cases, tiny air bubbles will be again trapped inside this polymeric viscous solution/dispersion. The incorporation of a foam reducing agent into the film-forming composition can reduce or eliminate the formation of air bubbles within the film.

The matrix of the present invention is formed into a sheet (wet film) prior to heating and drying. After the desired components are combined to form a multi-component matrix including the polymer and the solvent, and an active ingredient or other components as desired, the combination is formed into a wet film by any method known in the art such as extrusion, coating, spreading, casting or drawing the matrix. If a multi-layered film is desired, this may be accomplished by co-extruding more than one combination of components which may be of the same or different composition. A multi-layered wet film may also be achieved by coating, spreading, or casting a matrix onto an already formed film layer.

Although a variety of different film-forming techniques may be used, it is desirable to select a method that will provide a flexible film, such as reverse roll coating. The flexibility of the film allows for the sheets of film to be rolled and transported for storage or prior to being cut/divided into individual dosage forms. Desirably, the film will also be self-supporting or in other words able to maintain its integrity and structure in the absence of a separate support.

Coating or casting methods are particularly useful for the purpose of forming the films of the present invention. Specific examples include reverse roll coating, gravure coating, immersion or dip coating, metering rod or Meyer Bar coating, slot die or extrusion coating, gap or knife over roll coating, air knife coating, curtain coating, or combinations thereof.

Roll coating, or more specifically reverse roll coating, is particularly suitable. This procedure provides excellent control and uniformity of the resulting films. In this procedure, the coating material is measured onto the applicator roller by the precision setting of the gap between the upper metering roller and the application roller below it. The coating is transferred from the application roller to the substrate as it passes around the support roller adjacent to the application roller. Both three roll and four roll processes are common.

The gravure coating process relies on an engraved roller running in a coating bath, which fills the engraved dots or lines of the roller with the coating material. The excess coating on the roller is wiped off by a doctor blade and the coating is then deposited onto the substrate as it passes between the engraved roller and a pressure roller. Offset gravure is common, where the coating is deposited on an intermediate roller before transfer to the substrate.

In the metering rod coating process, an excess of the coating is deposited onto the substrate as it passes over the bath roller. The wire-wound metering rod, sometimes known as a Meyer Bar, allows the desired quantity of the coating to remain on the substrate. The quantity is determined by the diameter of the wire used on the rod.

In the slot die process, the coating is squeezed out by gravity or under pressure through a slot and onto the substrate. If the coating is solid, the process is termed "extrusion" and in this case, the line speed is frequently much faster than the speed of the extrusion. This enables coatings to be considerably thinner than the width of the slot.

It may be particularly desirable to employ extrusion methods for forming film compositions containing PEO (polyethylene oxide) polymer components. These compositions contain PEO or PEO blends (blends of polyethylene oxide polymers of different molecular weight) in the polymer component, and may be essentially free of added plasticizers, and/or surfactants, and polyalcohols.

The gap or knife over roll process relies on a coating being applied to the substrate which then passes through a "gap" between a "knife" and a support roller. As the coating and substrate pass through, the excess is scraped off. Air knife coating is where the coating is applied to the substrate and the excess is "blown off" by a powerful jet from the air knife. This procedure is useful for aqueous coatings. In the curtain coating process, a bath with a slot in the base allows a continuous curtain of the coating to fall into the gap between two conveyors. The object to be coated is passed along the conveyor at a controlled speed and so receives the coating on its upper face.

When a controlled or rapid drying process is desired, this may be achieved by a variety of methods. In the drying step, the solvents are preferably removed from the film in a manner such that the uniformity, or more specifically, the non-self-aggregating uniform heterogeneity, that is obtained in the wet film is maintained. Desirably, the film is dried from the bottom of the film to the top of the film. It is preferred that substantially no air flow is present across the top of the wet film during its initial setting period, during which a solid, visco-elastic structure is formed. This can take place within the first few minutes, e.g. the first 0.5 to 4 minutes of the drying process. Controlling the drying in this manner prevents the destruction and reformation of the film's top surface and the formation of bubbles.

The length of drying time can be properly controlled balanced with the heat sensitivity and volatility of the components, and particularly the flavor oils and drugs. The amount of energy, temperature and length and speed of the dryer can be balanced to accommodate such active ingredients and to minimize loss, degradation or ineffectiveness in the final film. Another method of controlling the drying process involves a zone drying procedure. A zone drying apparatus may include a continuous belt drying tunnel having multiple drying zones located within. The conditions of each drying zone may vary, for example, temperature and humidity may be selectively chosen. It may be desirable to sequentially order the zones to provide a stepped up drying effect.

### Examples

The effects of the present invention are illustrated by means of the following Examples.

### Example 1

A suspension was produced by mixing Buprenorphine HCl (15.53 g), Naloxone HCl dihydrate (4.41 g), lemon oil (1.36 g) and acetone (110 g). Further, an excipient solution was produced by mixing anhydrous trisodium citrate (2.44 g), citric acid monohydrate (5.36 g), acesulfam potassium (3.03 g), malitol solution (50% by weight, 3.51 g) as well as FD&C Yellow no. 6 (0.03 g) into 55 g of purified water.

Upon stirring of the suspension, the excipient solution was slowly added at room temperature. After completion of the addition of the excipient solution, stirring was continued while adding polyethylene oxide (MW: 100000 g/mol, 31.35 g), polyethylene oxide (MW: 200000 g/mol, 7.32 g), polyethylene oxide (MW: 900000 g/mol, 1.81 g) and hypromellose (10.83 g).

Stirring was then continued to prepare a homogeneous coating suspension (matrix). The matrix had a dynamic viscosity of 2500 mPa*s at 20°C, as measured using a conus/plate disc system (Manufacturer: Anton Paar Germany GmbH; Model: MCR 300; conus number CP 50/2 (50mm Diameter and 2° cone angle) and an agitation of 100 1/s.

The matrix is extruded onto a process liner (transporting support) to produce a wet film having a thickness of 700 µm and subsequently transported through a drying apparatus having the following constitution:
- Heating zone comprising an IR Dryer (Manufacturer: Mathis AG ; Model: IR field) positioned 30 cm above the process liner.
- Separation wall provided with heat insulation and having a slit of 7 cm in height for separating the heating zone from the drying zone and to allow transmission of the process liner supporting the heated wet film.
- Drying zone employing a hot air dryer using hot air of 50 °C, wherein 2/3 of the air flow were provided from below the transporting belt and 1/3 of the air flow was provided from above the transporting belt.

The IR dryer was driven at a power ratio of 3% which corresponds to a total IR power emitted towards the transporting belt of 4.5 kW/m². The heating time was set to 60 seconds. At the end of the heating zone, the wet film had a surface temperature of 30 °C.

Hot air drying was performed for 900 seconds. After leaving the drying zone, the resulting ingestible film was cut into sub-units of 2.8 cm². A group of 10 sub-units was randomly selected and analyzed for its residual acetone content by head space GC (gas chromatograph with a headspace sampler). The results are shown in Table 1. No formation of bubbles could be recognized with the bare eye.

### Examples 2 and 3

Except for changing the IR power ratio to 6% (corresponding to 9 kW/m²) and 9% (corresponding to 13.5 kW/m²), respectively, sub-units of an ingestible film were produced by the procedure described in Example 1. The results are shown in Table 1. No formation of bubbles could be recognized with the bare eye.

### Comparative Example 1

Except for switching off the IR dryer, sub-units of an ingestible film were produced by the procedure described in Example 1. The results are shown in Table 1. The ingestible films showed bubbles and had an unpleasant appearance.

**Table 1:**

| | Heating/Drying procedure | IR power | Temp. of heated wet film | Residual acetone |
|---|---|---|---|---|
| Example 1 | IR + hot air | 3% | 30 °C | 25199 ppm |
| Example 2 | IR + hot air | 6% | 40 °C | 9473 ppm |
| Example 3 | IR + hot air | 9% | 50 °C | 7937 ppm |
| C-Ex. 1 | hot air | --- | 15 °C | 39889 ppm |

As can be seen from Table 1, even a relatively low pre-heating power (3% IR power ratio) which results in a temperature of 30 °C at the end of the heating zone leads to a decrease of more than 60% in the amount of residual acetone. When raising the IR power, a further decrease of more than 50% can be achieved due to the higher temperature at the end of the heating zone (cf. Example 2). A further increase of the IR power leads to no significant further reduction in the residual acetone content (cf. Example 3). When no IR power is used for heating, the temperature of the wet film drops below room temperature due to evaporation of acetone (and water).

## Claims

1. A method for manufacturing an ingestible film, the method comprising:
providing a matrix comprising at least a solvent and a polymer;
a film forming step of forming a wet film from said matrix;
a heating step of heating said wet film by radiation originating from a radiation source to obtain a heated wet film having a temperature of at least 22°C; and
a drying step of drying said heated wet film using a drying means.

2. The method according to claim 1, wherein said radiation is IR radiation.

3. The method according to claim 1 or 2, wherein said radiation source is provided above the wet film.

4. The method according to claim 1 to 3, wherein said wet film is heated by said radiation to a temperature of at least 25°C, preferably at least 40°C, preferably not more than 60°C, more preferably not more than 55°C, before said drying step.

5. The method according to any one of claims 1 to 4, wherein said wet film is heated by said radiation for 0.1 to 10 minutes before said drying step.

6. The method according to any one of claims 1 to 5, wherein said drying means in said drying step is an oven and/or hot air.

7. The method according to any one of claims 1 to 6, wherein said wet film is dried at a drying temperature of 100°C or less, preferably 90°C or less, further preferably 80°C or less, preferably 30°C or more, preferably 50°C or more, preferably 70°C or more, in said drying step.

8. The method according to any one of claims 1 to 7, wherein said matrix further comprises a pharmaceutically active ingredient.

9. An ingestible film formed from a matrix comprising at least a solvent and a polymer, the ingestible film being obtainable by a method comprising the steps of
forming a wet film from said matrix,
heating said wet film by radiation originating from a radiation source to obtain a heated wet film having a temperature of at least 22°C; and
drying said heated wet film using drying means.

10. The ingestible film according to claim 9, further comprising an active ingredient.

11. A pharmaceutical dosage form comprising the ingestible film according to claim 9 or 10.

12. An apparatus for drying a wet film, the apparatus comprising at least
a transporting support for transporting said wet film;
a radiation zone comprising a radiation source for heating said wet film; and
a drying zone comprising a drying means for drying said heated wet film.

13. The apparatus according to claim 12, wherein said drying means is provided in addition to said radiation source.

14. The apparatus according to claim 12 or 13, wherein said apparatus is adapted to transport said wet film through said radiation zone and subsequently through said drying zone.

15. The apparatus according to any one of claims 12 to 14, which is adapted to carry out the method according to any one of claims 1 to 8.
